# EUROPÄISCHE PATENTSCHRIFT

(11) **EP 3 297 700 B1**
(45) Veröffentlichungstag und Bekanntmachung des Hinweises auf die Patenterteilung: **20.03.2019**
(21) Anmeldenummer: 16724288.2
(22) Anmeldetag: 19.05.2016
(51) Int. Cl.: A61M 1/16, F28D 21/00

(54) **BLUTBEHANDLUNGSGERÄT**
BLOOD TREATMENT DEVICE
APPAREIL DE TRAITEMENT DU SANG

(30) Priorität: 21.05.2015 DE 102015006601
(43) Veröffentlichungstag der Anmeldung: 28.03.2018
(73) Patentinhaber: Fresenius Medical Care Deutschland GmbH, 61352 Bad Homburg (DE)
(72) Erfinder: SPICKERMANN, Reiner, 97535 Burghausen (DE); BALSCHAT, Klaus, 97525 Schwebheim (DE); STÄBLEIN, Tilman, 97072 Würzburg (DE); PETERS, Arne, 61352 Bad Homburg (DE); KLÖFFEL, Peter, 97720 Nüdlingen (DE)
(74) Vertreter: Herrmann, Uwe
(86) Internationale Anmeldenummer: PCT/EP2016/000835
(87) Internationale Veröffentlichungsnummer: WO 2016/184572

(56) Entgegenhaltungen:
- WO-A1-2010/040819
- WO-A1-2012/053958
- DE-A1-102007 041 766

## Beschreibung

Die vorliegende Erfindung betrifft ein Blutbehandlungsgerät mit wenigstens einem Wärmetauscher, der zumindest einen ersten Raum und zumindest einen zweiten Raum aufweist, wobei im Betrieb des Blutbehandlungsgerätes der erste Raum von wenigstens einem ersten Fluid und der zweite Raum von wenigstens einem zweiten Fluid durchströmt wird und wobei der Wärmetauscher zumindest eine Membran aufweist, die den genannten ersten Raum von dem genannten zweiten Raum trennt.

WO 2010/040819 A1 offenbart einen Blutbehandlungsgerät für Nierenersatztherapie mit einem Wärmetauscher, der einen ersten Raum, einen zweiten Raum und eine Membran aufweist, die den ersten von dem zweiten Raum trennt.

Die Erfindung bezieht sich somit auf ein Blutbehandlungsgerät, insbesondere auf ein Dialysegerät, mit zumindest einem Wärmetauscher bzw. Wärmeübertrager (Rekuperator). Der Wärmetauscher weist wenigstens zwei durch zumindest eine wärmeübertragenden Membran voneinander getrennte Medienräume auf.

Vor dem Hintergrund, die Wahrscheinlichkeit einer Kontamination des ersten Fluids durch das zweite Fluid oder umgekehrt zu minimieren, besteht die Aufgabe der vorliegenden Erfindung darin, ein Blutbehandlungsgerät der eingangs genannten Art dahingehend weiter zu bilden, dass eine mögliche Leckage durch die Membran und somit eine hydraulische Verbindung der beiden Medienräume des Wärmetauschers schnell und zuverlässig erkannt wird.

Diese Aufgabe wird durch ein Blutbehandlungsgerät mit den Merkmalen des Anspruchs 1 gelöst.

Danach ist vorgesehen, dass die Membran einen Bestandteil zumindest eines Kondensators bildet, der wenigstens zwei Kondensatorplatten aufweist, zwischen denen sich die wenigstens eine Membran befindet.

Des Weiteren sind mit dem Kondensator in Verbindung stehende Überwachungsmittel vorgesehen, die so ausgebildet sind, dass diese wenigstens eine elektrische oder physikalische Eigenschaft des Kondensators zum Zwecke der Erfassung der Leckage von dem ersten zu dem zweiten Raum erfassen.

Der vorliegenden Erfindung liegt somit der Gedanke zugrunde, eine mögliche Leckage durch die Membran und somit eine hydraulische Verbindung der beiden Medienräume, d.h. des ersten und des zweiten Raums, die zu einer Kontamination der Fluide führen kann, durch eine elektrische Überwachung zu detektieren.

Diese elektrische Überwachung erfolgt dadurch, dass der Kondensator auf eine oder mehrere seiner elektrischen oder physikalischen Eigenschaften, wie beispielsweise der Impedanz, der Kapazität, einem an dem Kondensator gemessenen Ausgangssignal, wie z.B. einer Spannung etc. überwacht wird.

Wird durch die Überwachungsmittel eine Abweichung von einem Soll-Wert oder Soll-Verlauf des betreffenden Parameters festgestellt, kann auf eine Leckage geschlossen werden und es können entsprechende Gegenmaßnahmen ergriffen werden.

Durch diese elektrische Überwachung des Kondensators bzw. der Membran geht die Wahrscheinlichkeit der Verabreichung einer kontaminierten Flüssigkeit zum Beispiel einer Versorgungsflüssigkeit durch biologische sowie chemische Stoffe gegen Null.

Vorzugsweise arbeiten die Überwachungsmittel kontinuierlich, d.h. es erfolgt während des Betriebes des Blutbehandlungsgerätes eine durchgehende Überwachung. Von der Erfindung ist jedoch auch jede andere Art der Überwachung, wie z.B. eine Überwachung nach Ablauf bestimmter Zeitintervalle oder zu bestimmten Zeitpunkten erfasst.

Durch die Überwachung der Membran des Wärmetauschers kann ggf. auf andere, risikosenkende Gegenmaßnahmen verzichtet werden, da das Blutbehandlungsgerät unmittelbar und ohne die Auswertung weiterer Überwachungseinrichtungen auf eine detektierte Leckage reagieren kann. Vorzugsweise ist somit vorgesehen, dass weitere Mittel zur Leckageüberwachung nicht vorhanden sind.

Denkbar ist eine Anordnung, bei der sich sowohl die Kondensatorplatten als auch die Membran zwischen dem ersten und dem zweiten Raum befinden. In diesem Fall befinden sich die Kondensatorplatten und die Membran somit zwischen den beiden Fluiden und eine Leckage in einer oder beider der Kondensatorplatten und/oder der Membran kann detektiert werden.

An dieser Stelle wird darauf hingewiesen, dass der Begriff "Kondensatorplatten" jedes beliebige Element umfasst, das als Kondensatorbestandteil verwendet werden kann, unabhängig davon, ob das Element plattenförmig ist oder eine andere Form aufweist.

Von der Erfindung ist auch der Fall umfasst, dass sich die Membran zwischen dem ersten und dem zweiten Raum befindet und dass der erste und der zweite Raum zwischen den Kondensatorplatten angeordnet sind.

Somit wird insgesamt ein Kondensator ausgebildet, in dessen Innenraum, d.h. zwischen dessen Platten nicht nur die Membran angeordnet ist, sondern auch das erste und zweite Fluid. Somit wird ein Dielektrikum ausgebildet, dessen Eigenschaften sich im Fall einer Leckage aufgrund des Flüssigkeitsübertritts zwischen Primär- und Sekundärseite, d.h. von dem ersten in den zweiten Raum oder umgekehrt verändert.

Die Überwachungsmittel können eine Gleich- oder Wechselspannungsquelle umfassen, deren Pole mit den Kondensatorplatten verbunden sind. Um eine sich bei einer Leckage ändernde elektrische Eigenschaft des Kondensators zu erfassen, kann die Spannung über dem Kondensator erfasst werden und aus einer Änderung z.B. der Amplitude, der Frequenz oder einer Phasenverschiebung der gemessenen Spannung gegenüber der Eingangsspannung oder gegenüber der Spannung im Sollzustand (d.h. ohne Leckage) rückgeschlossen werden, dass eine Leckage eingetreten ist.

Grundsätzlich ist jedoch auch die Verwendung einer Gleichspannungsquelle denkbar. In diesem Fall wäre es z.B. möglich, den ohmschen Widerstand des Kondensators zu messen und bei einer Widerstandsänderung auf eine Leckage zu schließen.

Die beiden Kondensatorplatten können unmittelbar und beidseitig an der Membran anliegen, sodass sich ein sandwichartiger Aufbau ergibt.

Die Kondensatorplatten bestehen vorzugsweise aus Metall und insbesondere aus rostfreiem Stahl oder Titan.

Die Membran ist vorzugsweise als elektrischer Isolator, jedoch wärmeleitend ausgeführt, sodass ein guter Wärmeübergang zwischen den beiden Räumen des Wärmetauschers möglich ist.

Denkbar ist es, die Membran aus Kapton auszuführen. Bei diesem Material handelt es sich um ein Polyimid. Grundsätzlich können für die Membran auch andere Polymere bzw. andere Materialien verwendet werden.

Grundsätzlich kommen als durch die Überwachungsmittel überwachte Parameter beliebige elektrische oder physikalische Eigenschaften des Kondensators in Frage, die sich in Falle einer Leckage ändern. Als überwachte Parameter sind beispielsweise die Impedanz oder die Kapazität des Kondensators denkbar.

Auch ist es denkbar, dass der Kondensator in einen Schwingkreis integriert ist und dass die Überwachungsmittel so ausgebildet sind, dass diese eine Messung der Resonanzfrequenz des Kondensators durchführen.

Ändert sich die Impedanz, die Kapazität oder auch die Resonanzfrequenz des Kondensators, kann auf eine Leckage der Membran und/oder der Kondensatorplatte(n) geschlossen werden.

Denkbar ist es, dass die Überwachungsmittel zumindest einen Messwiderstand umfassen, über dem die Spannung ermittelt wird.

Dieser Messwiderstand kann eine Reihe zu dem durch die Membran gebildeten Widerstand geschaltet sein.

In einer weiteren Ausgestaltung der Erfindung weisen die Überwachungsmittel einen Tiefpassfilter zur Bestimmung eines Mittelwertes der über dem Kondensator gemessenen Spannung auf.

Des Weiteren kann vorgesehen sein, dass zum Zwecke der Überprüfung der Überwachungsmittel zumindest ein Testwiderstand vorgesehen ist bzw. einsetzbar ist, der parallel zu dem durch die Membran gebildeten Widerstand geschaltet ist oder werden kann.

Dieser Testwiderstand simuliert somit den Eintritt einer Leckage. Dementsprechend kann geprüft werden, ob die Überwachungsmittel bei eingesetztem Testwiderstand eine entsprechende Signaländerung ermitteln. Ist dies der Fall, kann auf die korrekte Funktionsweise der Überwachungsmittel geschlossen werden.

Vorzugsweise handelt es sich bei dem Blutbehandlungsgerät um ein Dialysegerät, wie beispielsweise um ein Hämodialysegerät.

Das Blutbehandlungsgerät kann mit einer Wasserversorgung in Verbindung stehen, wobei der Wärmetauscher so mit der Wärmeversorgung verbunden sein kann, dass der genannte erste Raum des Wärmetauschers durch Frischwasser, insbesondere durch RO-Wasser aus der Wasserversorgung durchströmt wird.

Dieses Frischwasser bzw. RO-Wasser kann dazu herangezogen werden, durch Zugabe von Konzentraten eine Dialyselösung herzustellen.

Der zweite Raum des Wärmetauschers kann so angeordnet sein, dass dieser von verbrauchter, erwärmter Dialyselösung durchströmt wird. Somit tritt ein Wärmeübergang von der verbrauchten Dialyselösung auf das Frischwasser ein.

Der Wirkungsgrad von Wärmetauschern steigt unter anderem mit zunehmender Temperaturdifferenz zwischen Primär- und Sekundärseite, d.h. zwischen den beiden Räumen des Wärmetauschers. In Dialysegeräten befindet sich der Punkt mit der kältesten Medientemperatur unmittelbar nach dem Wasseranschluss bzw. nach der Wasserzulaufstrecke. Wie ausgeführt, kann die Sekundärseite, d.h. der andere Raum mit aufgeheiztem Dialysat durchströmt werden, bevor dieses das Gerät in Richtung des Abflusses verlässt.

Durch die vorliegende Erfindung ist es möglich, insbesondere bei einer kontinuierlichen oder auch in zeitlichen Abständen durchgeführten Überwachung der wärmetauschenden Membran unmittelbar auf eine Leckage zu reagieren, beispielsweise indem Pumpen gestoppt oder Ventile geschlossen werden. Da eine derartige unmittelbare Reaktion ohne weiteres möglich ist, kann ggf. auf andere risikosenkende und kostenverursachende Gegenmaßnahmen verzichtet werden.

Die Überwachungsmittel können mit einer Anzeige in Verbindung stehen, an der der seitens der Überwachungsmittel erfasste Zustand angezeigt wird. Auch andere Ausgabemittel, wie beispielsweise akustische und/oder optische Mittel zur Information des Nutzers über das Auftreten einer Leckage sind denkbar und von der Erfindung mit umfasst.

Weitere Einzelheiten und Vorteile der Erfindung werden anhand eines in der Zeichnung dargestellten Ausführungsbeispiels näher erläutert.

Es zeigen:
- Figur 1:: Eine schematische Ansicht des Wasserzulaufs, des Dialysatablaufes und des Wärmetauschers ,
- Figur 2:: Eine erste Variante einer direkten elektrischen Überwachung der Membran,
- Figur 3:: Eine weitere Realisierungsmöglichkeit der elektrischen Membranüberwachung,
- Figur 4:: Eine Figur 2 entsprechende direkte elektrische Überwachung der Membran mit Testwiderstand,
- Figur 5:: Ein Schaltbild der Anordnung gemäß Figur 2 mit Messwiderstand und Tiefpassfilter zur Spannungswertermittlung,
- Figur 6:: Eine schematische Ansicht eines Kondensators ohne und mit Leckage und
- Figur 7:: Ein Schaltbild der Anordnung gemäß Figur 2 bei eingetretener Leckage.

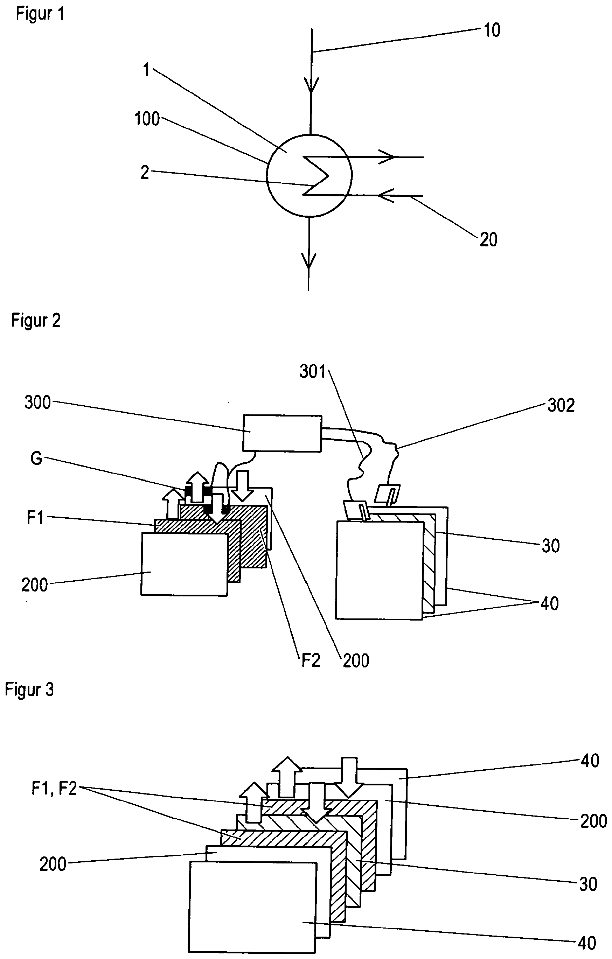

In Figur 1 ist mit dem Bezugszeichen 10 der Frischwasserzulauf eines Dialysegerätes dargestellt. Bei diesem Frischwasser handelt es sich vorzugsweise um RO-Wasser. Der erste Raum des Wärmetauschers 100 wird durch dieses Wasser durchströmt und der schematisch dargestellte zweite Raum 2 wird durch verbrauchtes Dialysat durchströmt, das durch die Leitung 20 in den zweiten Raum 2 einströmt.

Auf diese Weise erfolgt eine Erwärmung des RO-Wassers durch verbrauchtes Dialysat.

Zwischen den beiden Räumen 1 und 2 befindet sich eine wärmeleitende und elektrische isolierende Membran, die in den folgenden Figuren wiedergegeben ist.

Figur 2 zeigt diese Membran mit dem Bezugszeichen 30. Dabei handelt es sich um einen elektrischen Isolator, der beidseitig an metallische Lagen 40 als Kondensatorplatten angrenzt bzw. von diesen umgeben ist. Diese beiden metallischen Lagen können beispielsweise durch Titan- oder Stahlmembranen gebildet werden.

Eine Möglichkeit der Überwachung der Membran 30 besteht somit darin, dass die Membran von zwei Stahlmembranen bzw. Kondensatorplatten 40 umgeben ist.

Diese Gesamtanordnung aus der Membran 30 und den Platten 40 ist zwischen den beiden Fluidwegen F1 und F2 angeordnet, wobei es sich bei dem Fluid gemäß Fluidweg F1 um das in dem ersten Raum 1 befindliche Frischwasser und bei dem Fluid gemäß Fluidweg F2 um das in dem zweiten Raum 2 befindliche gebrauchte Dialysat handelt.

Das Bezugszeichen 200 kennzeichnet ein Gehäuse der Anordnung beispielsweise aus PPO (Polyphenylenoxid) und das Bezugszeichen G die Erdung der beiden Fluidpfade F1 und F2.

Mit dem Bezugszeichen 300 ist eine Wechselspannungsquelle gekennzeichnet, die über die Kabel 301 und 302 mit den Kondensatorplatten 40 in elektrischer Verbindung steht und diese mit einer Wechselspannung beaufschlagt.

Die Realisierungsmöglichkeit gemäß Figur 3 unterscheidet sich von der gemäß Figur 2 dadurch, dass die Kondensatorplatten 40 nicht zwischen den beiden Fluidpfaden F1 und F2 angeordnet sind, sondern diese einfassen. In anderen Worten sind die Fluidpfade F1 und F2 zwischen den beiden Kondensatorplatten 40 aufgenommen. Zwischen den Fluidpfaden F1 und F2 befindet sich der elektrische Isolator in Form der Membran 30.

Das Gehäuse wird auch gemäß Figur 3 durch das Bezugszeichen 200 gekennzeichnet.

In der Variante gemäß Figur 3 kommt somit ein elektrischer Isolator 30 mit hinreichender Wärmeleitung als interne Membran zum Einsatz. Durch Anbringen von elektrisch leitfähigen Platten 40 im Außenbereich erhält man einen Kondensator, dessen Innenraum mit einem Dielektrikum aufgeführt ist.

Im Falle einer internen Leckage über die Membran 30 kommt es zu einem Flüssigkeitsübertritt zwischen Primär- und Sekundärseite, d.h. zwischen den Fluidpfaden F1 und F2. Dieser Flüssigkeitsübertritt hat eine Veränderung der dielektrischen Eigenschaften zur Folge. Die resultierende Kapazitätsänderung kann gemessen und somit eine aufgetretene Leckage detektiert werden.

Somit wird auch gemäß Figur 3 insgesamt ein Kondensator ausgebildet, wobei im Unterschied zu der Ausführung gemäß Figur 2 Fluidpfade einen Bestandteil des Kondensators bilden.

Alle dargestellten Varianten beruhen auf dem Prinzip einer sich ändernden Impedanz bzw. Kapazität im Falle einer Leckage. Somit können sämtliche bekannten Möglichkeiten zur Messung dieser physikalischen Größen oder auch anderer elektrischer bzw. physikalischer Eigenschaften des Kondensators herangezogen werden, um auf eine Leckage zurückschließen zu können.

Dazu zählt beispielsweise auch die Integration des Kondensators in einen Schwingkreis und die mit einer Leckage verbundene Verschiebung der Resonanzfrequenz.

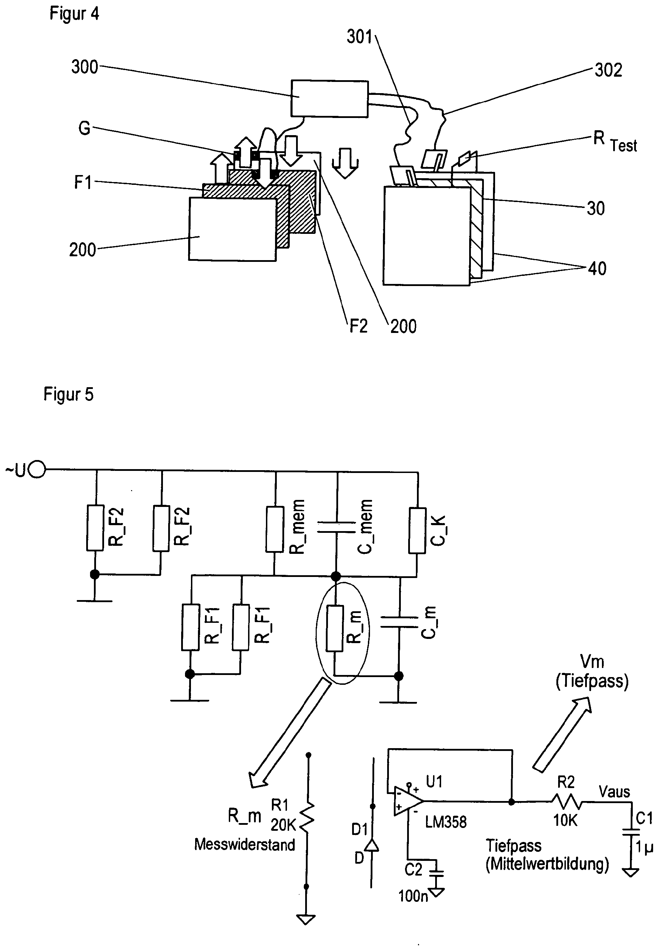

Figur 4 zeigt eine Figur 2 entsprechende Anordnung, bei der zusätzlich zwischen den beiden Kondensatorplatten 40 ein Testwiderstand geschaltet ist. Dieser Testwiderstand simuliert eine über die Membran 30 erfolgende Leckage und ermöglicht auf diese Weise eine korrekte Überprüfung des Ansprechens der Überwachungsmittel auf eine solche Leckage.

Die Überwachungsmittel können beispielsweise durch eine Messanordnung gebildet werden, die elektrisch mit den Kondensatorplatten 40 verbunden ist und die zwischen diesen auftretende Wechsel- oder Gleichspannung und/oder den Stromfluss erfasst.

Figur 5 zeigt ein Schaltbild der Anordnung gemäß Figur 2, wobei es sich bei der Größe R_F2 um den ohmschen Widerstand handelt, der durch das verbrauchte Dialysat gemäß F2 gebildet wird und bei der Größe R_F1 um den ohmschen Widerstand handelt, der durch die frische Lösung bzw. das zulaufende Wasser gemäß F1 gebildet wird.

Die Größe R_mem stellt den durch die Membran gebildeten ohmschen Widerstand dar und die Größe C_mem den durch die Kondensatorplatten 40 einschließlich der Membran 30 gebildete Kapazität des Kondensators dar.

Mit C_K ist die Kapazität der Leitungen 301 und 302, mit R_m der ohmsche Widerstand der Messanordnung bzw. der Überwachungsmittel und mit C_m deren Kapazität gekennzeichnet.

Wie dies aus Figur 5 hervorgeht, wird eingangsseitig eine Wechselspannung angelegt.

Eine Leckage oder das Zwischenschalten des Test-Widerstandes führt zu einer veränderten Amplitude der gemessenen Spannung und auch zu einer höheren Spannung über dem Messwiderstand, die durch Mittelwertbildung durch den Tiefpass gemäß Figur 5 ermittelt werden kann.

Dieser mittlere Spannungswert ist in Figur 5 mit Vm gekennzeichnet.

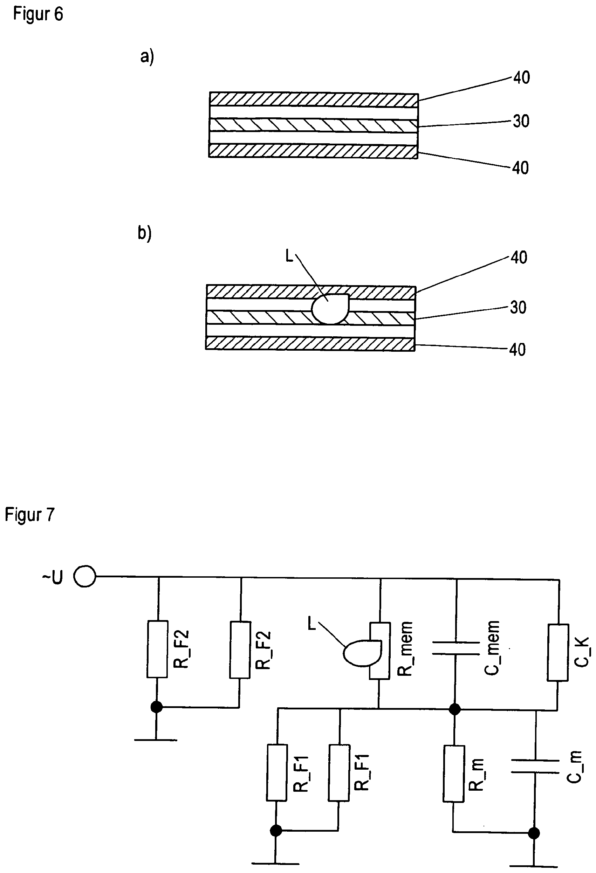

Figur 6 zeigt in schematischer Ansicht in der Abbildung a) die Membran 30, die sandwichartig von den beiden Kondensatorplatten 40 umgeben ist. In dem Zustand a) ist keine Leckage vorhanden.

Tritt eine Leckage auf, die die Kondensatorplatte 40 und die Membran 30 betrifft, wie dies aus Figur 6 b) hervorgeht, fließt ein erhöhter Strom durch den Messwiderstand R_m, wodurch die gemessene Spannung ansteigt.

Figur 7 zeigt die Schaltung gemäß Figur 5 mit eingetretener Leckage L.

Auf diese Weise ist unmittelbar der Eintritt eines Leckagefalles messbar, sodass entsprechende Gegenmaßnahmen ergriffen werden können. Die Überwachungsmittel können beispielsweise das Schließen eines oder mehrerer Ventile und/oder das Anhalten einer oder mehrerer Pumpen veranlassen.

Ein Test der Anordnung gemäß Figur 5 kann beispielsweise dadurch vorgenommen werden, dass eine höhere Sinusamplitude zu einem höheren Spannungswert an dem Messwiderstand R_m führt.

Die Sicherheit der erfindungsgemäßen Überwachung wird dadurch gewährleistet, dass bei einem Lösen der Kabel 301 oder 302 oder bei Ausbleiben der Spannungsversorgung ein entsprechend geringeres Signal gemessen wird.

Die Überwachung wenigstens einer physikalischen oder elektrischen Größe, die vorzugsweise kontinuierlich durch die Überwachungsmittel vorgenommen wird, ermöglicht es, im Falle einer Leckage an der Membran bzw. an den Kondensatorplatten die Änderung der Messgröße unmittelbar zu erfassen, wie beispielsweise die Impedanz oder die Kapazität des Kondensators.

Eine bevorzugte Ausgestaltung der Erfindung besteht darin, dass sich zwischen den beiden Räumen des Wärmetauschers bzw. Wärmeüberträgers der Kondensator befindet, der aus zumindest zwei Platten und wenigstens einem dazwischen angeordneten Isolator in Form der Membran besteht. In diesem Fall trennt der Kondensator die beiden Fluidwege bzw. den ersten und den zweiten Raum des Wärmetauschers voneinander.

## Patentansprüche

1. Blutbehandlungsgerät mit einem Wärmetauscher, der einen ersten Raum und einen zweiten Raum aufweist, wobei im Betrieb des Blutbehandlungsgerätes der erste Raum von einem ersten Fluid und der zweite Raum von einem zweiten Fluid durchströmt wird, und wobei der Wärmetauscher eine Membran aufweist, die den ersten von dem zweiten Raum trennt, **dadurch gekennzeichnet, dass** die Membran einen Bestandteil eines Kondensators bildet, der zwei Kondensatorplatten aufweist, zwischen denen sich die Membran befindet, und dass mit dem Kondensator in Verbindung stehende Überwachungsmittel vorgesehen sind, die derart ausgebildet sind, dass diese eine elektrische oder physikalische Eigenschaft des Kondensators zum Zwecke der Erfassung einer Leckage von dem ersten zu dem zweiten Raum erfassen.

2. Blutbehandlungsgerät nach Anspruch 1, **dadurch gekennzeichnet, dass** sich die Kondensatorplatten und die Membran zwischen dem ersten und dem zweiten Raum befinden.

3. Blutbehandlungsgerät nach Anspruch 1, **dadurch gekennzeichnet, dass** sich die Membran zwischen dem ersten und dem zweiten Raum befindet und dass der erste und der zweite Raum zwischen den Kondensatorplatten angeordnet ist.

4. Blutbehandlungsgerät nach einem der vorhergehenden Ansprüche, **dadurch gekennzeichnet, dass** die Überwachungsmittel eine Gleich- oder eine Wechselspannungsquelle umfassen, deren Pole mit den Kondensatorplatten verbunden sind.

5. Blutbehandlungsgerät nach einem der Ansprüche 1, 2 oder 4, **dadurch gekennzeichnet, dass** die Kondensatorplatten unmittelbar und beidseitig an der Membran anliegen, so dass sich ein sandwichartiger Aufbau ergibt.

6. Blutbehandlungsgerät nach einem der vorhergehenden Ansprüche, **dadurch gekennzeichnet, dass** die Kondensatorplatten aus Metall, insbesondere aus Stahl oder Titan bestehen und/oder dass die Membran als elektrischer Isolator ausgeführt ist und insbesondere aus Kapton besteht.

7. Blutbehandlungsgerät nach einem der vorhergehenden Ansprüche, **dadurch gekennzeichnet, dass** die Überwachungsmittel derart ausgebildet sind, dass diese eine Impedanzmessung oder eine Kapazitätsmessung des Kondensators durchführen.

8. Blutbehandlungsgerät nach einem der vorhergehenden Ansprüche, **dadurch gekennzeichnet, dass** der Kondensator in einen Schwingkreis integriert ist und das die Überwachungsmittel derart ausgebildet sind, dass diese eine Messung der Resonanzfrequenz des Kondensators durchführen.

9. Blutbehandlungsgerät nach einem der vorhergehenden Ansprüche, **dadurch gekennzeichnet, dass** die Überwachungsmittel einen Mess-Widerstand umfassen, über dem die Spannung ermittelt wird.

10. Blutbehandlungsgerät nach Anspruch 9, **dadurch gekennzeichnet, dass** der Mess-Widerstand in Reihe zu dem durch die Membran gebildeten Widerstand geschaltet ist.

11. Blutbehandlungsgerät nach Anspruch 9 oder 10, **dadurch gekennzeichnet, dass** die Überwachungsmittel einen Tiefpassfilter zur Bestimmung eines Mittelwertes der Spannung aufweisen.

12. Blutbehandlungsgerät nach einem der Ansprüche 9 bis 11, **dadurch gekennzeichnet, dass** zum Zwecke der Überprüfung der Überwachungsmittel ein Testwiderstand vorgesehen ist, der parallel zu dem durch die Membran gebildeten Widerstand geschaltet ist.

13. Blutbehandlungsgerät nach einem der vorhergehenden Ansprüche, **dadurch gekennzeichnet, dass** es sich bei dem Blutbehandlungsgerät um ein Dialysegerät handelt.

14. Blutbehandlungsgerät nach Anspruch 13, **dadurch gekennzeichnet, dass** das Blutbehandlungsgerät mit einer Wasserversorgung in Verbindung steht und dass der Wärmetauscher derart mit der Wasserversorgung verbunden ist, dass der erste Raum des Wärmetauschers durch Frischwasser, insbesondere mit RO-Wasser aus der Wasserversorgung durchströmt wird.

15. Blutbehandlungsgerät nach Anspruch 13 oder 14, **dadurch gekennzeichnet, dass** der zweite Raum des Wärmetauschers derart angeordnet ist, dass dieser von verbrauchter Dialyselösung durchströmt wird.

## Claims

1. A blood treatment device having a heat exchanger which comprises a first space and a second space, wherein, in the operation of the blood treatment device, the first space is flowed through by a first fluid and the second space is flowed through by a second fluid, and wherein the heat exchanger comprises a membrane which separates the first space from the second space, **characterized in that** the membrane forms a component of a capacitor which comprises capacitor plates between which the membrane is located, and **in that** monitoring means are provided which are connected to the capacitor, and which are configured such that they detect an electrical or physical property of the capacitor for the purpose of detecting a leak from the first space to the second space.

2. A blood treatment device in accordance with claim 1, **characterized in that** the capacitor plates and the membrane are located between the first space and the second space.

3. A blood treatment device in accordance with claim 1, **characterized in that** the membrane is located between the first space and the second space, and **in that** the first space and the second space are arranged between the capacitor plates.

4. A blood treatment device in accordance with one of the preceding claims, **characterized in that** the monitoring means comprise a DC or AC voltage source, the poles of which are connected to the capacitor plates.

5. A blood treatment device in accordance with one of claims 1, 2 or 4, **characterized in that** the capacitor plates contact the membrane directly and at both sides so that a sandwich-like structure results.

6. A blood treatment device in accordance with one of the preceding claims, **characterized in that** the capacitor plates consist of metal, in particular steel or titanium, and/or **in that** the membrane is configured as an electrical insulator and in particular consists of Kapton.

7. A blood treatment device in accordance with one of the preceding claims, **characterized in that** the monitoring means are configured such that they carry out an impedance measurement or a capacitance measurement of the capacitor.

8. A blood treatment device in accordance with one of the preceding claims, **characterized in that** the capacitor is integrated into a resonant circuit, and **in that** the monitoring means are configured such that they carry out a measurement of the resonant frequency of the capacitor.

9. A blood treatment device in accordance with one of the preceding claims, **characterized in that** the monitoring means comprise a measurement resistor over which the voltage is determined.

10. A blood treatment device in accordance with claim 9, **characterized in that** the measurement resistor is connected in series with the resistor formed by the membrane.

11. A blood treatment device in accordance with claim 9 or claim 10, **characterized in that** the monitoring means have a low pass filter for determining an average value of the voltage.

12. A blood treatment device in accordance with one of the claims 9 to 11, **characterized in that** a test resistor is provided for the purpose of checking the monitoring means, which is connected in parallel with the resistor formed by the membrane.

13. A blood treatment device in accordance with one of the preceding claims, **characterized in that** the blood treatment device is a dialysis device.

14. A blood treatment device in accordance with claim 13, **characterized in that** the blood treatment device is connected to a water supply, and **in that** the heat exchanger is connected to the water supply such that the first space of the heat exchanger is flowed through by fresh water, in particular by RO water from the water supply.

15. A blood treatment device in accordance with claim 13 or 14, **characterized in that** the second space of the heat exchanger is arranged such that it is flowed through by consumed dialysis solution.

## Revendications

1. Appareil de traitement du sang avec un échangeur thermique qui comporte un premier compartiment et un deuxième compartiment, lorsque l'appareil de traitement du sang est en marche, le premier compartiment étant traversé par un premier fluide et le deuxième compartiment par un deuxième fluide, et l'échangeur thermique comportant une membrane qui sépare le premier compartiment du deuxième, **caractérisé en ce que** la membrane fait partie intégrante d'un condensateur qui présente de deux plaques entre lesquelles se trouve la membrane, et **en ce que** des moyens de surveillance, qui sont en contact avec le condensateur, sont prévus et conçus de telle sorte qu'ils enregistrent une propriété électrique ou physique du condensateur aux fins de déceler une fuite du premier compartiment vers le deuxième.

2. Appareil de traitement du sang selon la revendication 1, **caractérisé en ce que** les plaques du condensateur et la membrane se trouvent entre le premier et le deuxième compartiments.

3. Appareil de traitement du sang selon la revendication 1, **caractérisé en ce que** la membrane se trouve entre le premier et le deuxième compartiments et **en ce que** le premier et le deuxième compartiments sont disposés entre les plaques du condensateur.

4. Appareil de traitement du sang selon l'une quelconque des revendications précédentes, **caractérisé en ce que** les moyens de surveillance comprennent une source de tension alternative ou continue dont les pôles sont reliés aux plaques du condensateur.

5. Appareil de traitement du sang selon l'une quelconque des revendications 1, 2 ou 4, **caractérisé en ce que** les plaques du condensateur restent directement et des deux côtés contre la membrane de telle sorte qu'une structure de type sandwich résulte.

6. Appareil de traitement du sang selon l'une quelconque des revendications précédentes, **caractérisé en ce que** les plaques du condensateur consistent en métal, en particulier en acier ou en titane, et/ou **en ce que** la membrane est conçue comme un isolateur électrique et notamment consiste en Kapton.

7. Appareil de traitement du sang selon l'une quelconque des revendications précédentes, **caractérisé en ce que** les moyens de surveillance sont réalisés de telle sorte qu'ils effectuent une mesure d'impédance ou de la capacité du condensateur.

8. Appareil de traitement du sang selon l'une quelconque des revendications précédentes, **caractérisé en ce que** le condensateur est intégré dans un circuit résonant et **en ce que** les moyens de surveillance sont réalisés de telle sorte que ceux-ci effectuent une mesure la fréquence de résonance du condensateur.

9. Appareil de traitement du sang selon l'une quelconque des revendications précédentes, **caractérisé en ce que** les moyens de surveillance comprennent une résistance de mesure via laquelle la tension est définie.

10. Appareil de traitement du sang selon la revendication 9, **caractérisé en ce que** la résistance de mesure est branchée en série avec la résistance formée par la membrane.

11. Appareil de traitement du sang selon la revendication 9 ou 10, **caractérisé en ce que** les moyens de surveillance comportent un filtre passe-bas afin de déterminer une valeur moyenne de la tension.

12. Appareil de traitement du sang selon l'une quelconque des revendications 9 à 11, **caractérisé en ce qu'**une résistance d'essai est prévue dans le but de vérifier les moyens de surveillance, et ladite résistance d'essai est branchée en parallèle avec la résistance formée par la membrane.

13. Appareil de traitement du sang selon l'une quelconque des revendications précédentes, **caractérisé en ce que** l'appareil de traitement du sang est une machine de dialyse.

14. Appareil de traitement du sang selon la revendication13, **caractérisé en ce que** l'appareil de traitement du sang communique avec une alimentation en eau et **en ce que** l'échangeur thermique est relié à l'alimentation en eau de telle sorte que le premier compartiment de l'échangeur thermique est traversé par de l'eau fraîche, en particulier de l'eau d'osmose inverse provenant de l'alimentation en eau.

15. Appareil de traitement du sang selon la revendication 13 ou 14, **caractérisé en ce que** le deuxième compartiment de l'échangeur thermique est disposé de telle sorte que celui-ci est traversé par une solution de dialyse usagée.
